# EUROPEAN PATENT SPECIFICATION

(11) **EP 2 953 649 B1**
(45) Date of publication and mention of the grant of the patent: **04.11.2020**
(21) Application number: 14709425.4
(22) Date of filing: 06.02.2014
(51) Int. Cl.: A61K 49/00, A61P 35/00

(54) **GROUNDBREAKING PLATFORM TECHNOLOGY FOR SPECIFIC BINDING TO NECROTIC CELLS**
BAHNBRECHENDE PLATTFORMTECHNOLOGIE ZUR SPEZIFISCHEN BINDUNG AN NEKROTISCHE ZELLEN
TECHNOLOGIE RÉVOLUTIONNAIRE DE PLATE-FORME POUR LA LIAISON SPÉCIFIQUE À DES CELLULES NÉCROTIQUES

(30) Priority: 06.02.2013 WO PCT/NL2013/050064; 06.02.2013 WO PCT/NL2013/050067; 06.08.2013 NL 2011274
(43) Date of publication of application: 16.12.2015
(73) Proprietor: HQ Medical (Netherlands) B.V., 3043 NA Rotterdam (NL)
(72) Inventor: MARING, Markwin, NL-3400 AC Rotterdam (NL); LÖWIK, Clemens Waltherus Gerardus Maria, NL-3400 AC Rotterdam (NL); VAN BEEK, Ermond R., NL-3400 AC Rotterdam (NL); BANGWEN, Xie, NL-3400 AC Rotterdam (NL)
(74) Representative: Vogels, Leonard Johan Paul
(86) International application number: PCT/NL2014/050074
(87) International publication number: WO 2014/123418

(56) References cited:
- WO-A1-2013/119111
- WO-A1-2013/119114
- US-A1- 2005 014 216
- US-A1- 2012 088 262
- MADHURI DASARI ET AL: "Hoechst-IR: An Imaging Agent That Detects Necrotic Tissue in Vivo by Binding Extracellular DNA", ORGANIC LETTERS, vol. 12, no. 15, 6 August 2010 (2010-08-06), pages 3300-3303, XP055092139, ISSN: 1523-7060, DOI: 10.1021/ol100923d cited in the application
- Bryan A Smith ET AL: "IN VIVO OPTICAL IMAGING OF CELL DEATH USING FLUORESCENT SYNTHETIC COORDINATION COMPLEXES", , 1 April 2012 (2012-04-01), XP055092601, Retrieved from the Internet: URL:http://etd.nd.edu/ETD-db/theses/availa ble/etd-04132012-141111/unrestricted/Smith Bryan042012D.pdf [retrieved on 2013-12-10] cited in the application
- EPSTEIN A L ET AL: "A NOVEL METHOD FOR THE DETECTION OF NECROTIC LESIONS IN HUMAN CANCERS", CANCER RESEARCH, AMERICAN ASSOCIATION FOR CANCER RESEARCH, US, vol. 48, no. 20, 15 October 1988 (1988-10-15), pages 5842-5848, XP008062317, ISSN: 0008-5472 cited in the application
- VERA D R ET AL: "Cy5.5-DTPA-galactosyl-dextran: a fluorescent probe for in vivo measurement of receptor biochemistry", NUCLEAR MEDICINE AND BIOLOGY, ELSEVIER, NY, US, vol. 32, no. 7, 1 October 2005 (2005-10-01), pages 687-693, XP027674804, ISSN: 0969-8051 [retrieved on 2005-10-01] cited in the application
- UZGIRIS EGIDIJUS E ET AL: "A multimodal contrast agent for preoperative MR Imaging and intraoperative tumor margin delineation", TECHNOLOGY IN CANCER RESEARCH AND TREATMENT, ADENINE PRESS, SCHENECTADY, NY, US , vol. 5, no. 4 1 August 2006 (2006-08-01), pages 301-309, XP008106889, ISSN: 1533-0346 Retrieved from the Internet: URL:http://www.tcrt.org/index.cfm?d=3025&c =4209&p=15144&do=detail cited in the application
- LIU T ET AL: "A targeted low molecular weight near-infrared fluorescent probe for prostate cancer", BIOORGANIC & MEDICINAL CHEMISTRY LETTERS, PERGAMON, GB, vol. 20, no. 23, 1 December 2010 (2010-12-01), pages 7124-7126, XP027459371, ISSN: 0960-894X, DOI: 10.1016/J.BMCL.2010.09.057 [retrieved on 2010-11-02] cited in the application
- FANG F ET AL: "Determination of nucleic acids with a near infrared cyanine dye using resonance light scattering technique", SPECTROCHIMICA ACTA. PART A: MOLECULAR AND BIOMOLECULAR SPECTROSCOPY, ELSEVIER, AMSTERDAM, NL, vol. 64, no. 3, 1 June 2006 (2006-06-01), pages 698-702, XP028036086, ISSN: 1386-1425, DOI: 10.1016/J.SAA.2005.07.071 [retrieved on 2006-06-01] cited in the application
- HONG ZHENG ET AL: "Dye-binding protein assay using a long-wave-absorbing cyanine probe", ANALYTICAL BIOCHEMISTRY, vol. 318, no. 1, 1 July 2003 (2003-07-01), pages 86-90, XP055092454, ISSN: 0003-2697, DOI: 10.1016/S0003-2697(03)00163-5 cited in the application
- VOLKOVA ET AL: "Cyanine dye-protein interactions: Looking for fluorescent probes for amyloid structures", JOURNAL OF BIOCHEMICAL AND BIOPHYSICAL METHODS, AMSTERDAM, NL, vol. 70, no. 5, 10 July 2007 (2007-07-10), pages 727-733, XP022144582, ISSN: 0165-022X, DOI: 10.1016/J.JBBM.2007.03.008 cited in the application
- HAIBIAO GONG ET AL: "Near-Infrared Fluorescence Imaging of Mammalian Cells and Xenograft Tumors with SNAP-Tag", PLOS ONE, vol. 7, no. 3, 30 March 2012 (2012-03-30), page e34003, XP055093071, ISSN: 1932-6203, DOI: 10.1371/journal.pone.0034003 cited in the application

## Description

### FIELD OF THE INVENTION

The invention relates to a system comprising a targeting molecule for binding necrotic cells; to the system for use as a medicament; to use of the system for diagnosis; to the system for use in treatment of diseases involving necrotic cell death, to a dosage comprising the system; to a method for determining localization of the composition within a sample using said system; and, to a method for treatment of cancers and/or diseases involving necrotic cell death using said system. In particular the invention relates to compositions targeting necrotic cells.

### BACKGROUND OF THE INVENTION

Prior art systems and methods involving targeting dead cells are not optimal and/or applicable:
**a. Necrotic vs. Apoptotic Cell Death**
   Targeting of necrosis is specific and unique because necrosis only appears in pathological conditions of cell death due to an insufficient blood supply and thus a lack of oxygen, trauma or due to direct cytotoxic agents or other cancer treatments like radiotherapy or photo dynamic therapy. This is in contrast to apoptotic cell death, which occurs continuously during tissue turnover. Therefore apoptotic cell death, in contrast to necrotic cell death, is not useable for targeting diseases characterized by necrosis as found for instance in case of tumours (rapidly growing tumours spontaneously develop necrotic cores), trauma, infarcts, osteoarthritis, diabetes, arteriosclerotic plaques, burns, certain bacterial infections, etc.
**b. Antibodies vs. Small molecules**
   Our compounds are small molecules, cyanine based dyes, which penetrate deeply into necrotic tissue. Antibodies have been used for targeting necrosis (Tumour Necrosis Targeting) but have the disadvantage that they are too large to penetrate in the necrotic tissue.
**c. Other Chemical Agents vs. Cyanine Dyes**
   There are other chemical agents (for instance PDT-Agents) which are known for labelling necrotic cells (for instance Hypericine, Bacteriochlorophyll derivatives, Gadophrin-2A and bis-DTPA-NI). These agents have specific characteristics which make them less suitable for imaging and therapy of above mentioned diseases while they are (1.) difficult to produce chemically, they are (2.)photo sensitive (the compound will generate oxygen radicals and change their properties in case of exposure to light),(3.) the clearance from the body takes much longer (up to several days) and (4.) the compounds are relatively large which makes it more difficult to penetrate in necrotic tissue. Cyanine dyes are easy to produce chemically, are photo stable, are rapidly cleared from the body (3 to 24 hours)and deeply and easily penetrate in necrotic tissue.

Further details on background may be obtained from previously filed applications PCT/NL2013/050067 and PCT/NL2013/050064.

The following documents may be considered:
Madhuri Dasari et al., Organic Letters, 2010, 12(15), 3300-3303, recite an imaging agent that detects necrotic tissue in vivo by binding extracellular DNA.

The present invention does not relate to DNA binding. DNA-binding molecules are generally considered unsuitable for use in humans due the high chance that such molecules are mutagenic/carcinogenic.

Bryan A Smith et al., URL:http://etd.nd.edu/ETD-db/theses/available/etd-04132012-141111/unrestricted/SmithBryan042012D.pdf, recites in vivo imaging of cell death using fluorescent synthetic coordination complexes. In an aspect, the document recites a number apoptosis targeting molecules coupled to a cyanine dye.

The cyanine in these complexes is solely used as fluorescent reporter.

Epstein A L et al., Cancer Research, 1988, 48(20), 5842-5848 relates to a method for the detection of necrotic lesions in human cancers. Such relies on using antibodies as targeting moiety coupled to a dye as reporter.

The production, storage and use of antibodies is very difficult, expensive and high risk in terms of adverse effects.

Vera D et al., Nuclear Medicine and Biology, 2005, 32, 687-693, relates to a fluorescent probe for in vivo measurement of receptor biochemistry.

Uzgiris E E et al., Technology in Cancer Research and Treatment, 2006, 5(4), 301-309, relates to a multimodal contrast agent for preoperative MR imaging and intraoperative tumor margin delineation.

Lia T et al, Bioorganic and Medicinal Chemistry Letters, 2010, 20, 7124-7126, relates to a low molecular weight near-IR probe for prostate cancer.

All of Vera D et al., Uzgiris E E et al and Lia T et al recite the use of cyanines as a reporter molecule coupled to various targeting moieties.

US 2012/088262 A1 relates to cyanine compounds, conjugates and methods for their use. Specifically said document recites amine-reactive cyanines.

It is noted that all proteins comprise amines. Amine reactive molecules will bind to any protein without large differences in affinity. In biological samples, such as blood and tissue, there is a vast amount of extracellular protein present. Selectivity for e.g. dead cells in the sample cannot therefore be achieved. In vivo, strong covalent binding to proteins will result in only very slow clearance from the body, which increases the chance of adverse effects.

Fang F et al., Spectrochimica Acta . Part A: molecular and biomolecular spectroscopy, 2006, 64(3), 698-702, relates to determination of nucleic acids with near IR cyanine dyes using light-scattering techniques. Affinity of cyanines for DNA and RNA is identified.

DNA binding agents have limited use in humans as previously identified.

Hong Zheng et al., Analytical Biochemistry, 2003, 318, 86-90, relates to a dye binding assay using a long-wave-absorbing cyanine probe and describes the interaction of dye SHMC with various proteins.

Only analytical applications are identified.

Volkova K D et al., Journal of Biochemical and Biophysical Chemisty, 2007, 70, 727-733 relates to fluorescent probes for amyloid structures and in particular to cyanines capable of binding aggregates of the amyloid-β peptide.

Binding results from an interaction between the dye and features of the amyloid-β aggregate, not with individual amyloid-β peptides. Amyloid-β aggregates are extracellular.

US 2005/014216 relates using rhodamines and phalloidin capable of binding intracellular proteins as probes for hepatotoxicity.

Haibiao Gong et al., PLoS One, 2012, 7(3), e34003, XP055093071, relates to relates to near IR fluorescence imaging of mammalian cells and xenograft tumors with SNAP-Tag.

This document describes only applications of click chemistry (in this case via SNAP-Tag).

It is an object of the present invention to overcome one or more disadvantages of the compositions of the prior art and to provide alternatives to current compositions for diagnosis and treatment of cancers and other diseases involving necrotic cell death, without jeopardizing functionality and advantages.

### SUMMARY OF THE INVENTION

The present invention describes a system comprising a targeting molecule for binding necrotic cells, and one or more of an agent, a vehicle and an in the body non-reactive molecule according to claim 1.

The present system comprises at least two entities, the entities being joined, such as by a chemical bond, each entity serving a distinct function within the system. It is noted more than one agent may be present, as well as more than one vehicle, and more than one non-reactive molecule. As such e.g. a combination therapy is feasible with the present system.

The present targeting molecule is very selective and very specific in binding to necrotic cells. As a result very low concentrations c.q. amounts of the present system may be used to provide advantageous effects thereof. It is noted that apoptosis specific probes, in contrast to necrosis specific probes will also target healthy tissue as apoptosis is involved in normal tissue turnover.

It has been found in earlier research by the present inventors that the targeting molecule specifically binds non-covalently to intracellular proteins such as actin, which are only available for the targeting molecule when the membrane integrity of a cell is lost, that is in case of a necrotic cell. For optimal targeting, the necrotic cells, are preferably in an early stage of necrosis, such as cells that have been dead for less than a few days, preferably less than half a day, such as a few hours, such as 2 hours, such as just dead cells. Characteristics of targeting molecules of the present invention, responsible for their effective targeting and/or safety, are that they are cell membrane impermeant, i.e. they cannot (significantly) cross the cell membrane of healthy cells; they are non-activated; they are capable of non-covalently binding to intracellular proteins (their target molecules); and, they are not capable/do not significantly bind to DNA (or RNA).

In case the system comprises an agent it may relate to an imaging compound, such as for use in an imaging technique, a therapeutic compound, in order to provide a therapeutic effect to necrotic cells or a part of a body (comprising necrotic cells) in need for such a therapeutic, such as a cancer, and a release factor, for releasing a further compound acting e.g. as a stimulus.

In case the system comprises a vehicle the vehicle functions as a transport medium for transporting an entity to necrotic cells, or as above a part of a body. As such a minute amount of entity can be target and transported to a desired location in a body. The vehicle may transport the present agent, it may transport a relative to the agent, different entity, and combinations thereof.

In case the system comprises an in the body non-reactive molecule it is intended to have this molecule interact with a counterpart. Interaction may (further) relate to one or more of activation by an activator, support by a catalyst, generation of a medicament upon interaction, and release of a medicament upon interaction. It is noted that from a functional point of view molecule and counterpart may be interchanged.

The system relates to non-toxic small molecules, having an ability to bind to necrotic cells and tissue. These molecules do not interact with DNA, i.e. these are not mutagenic. Typically the present system has a wide-spread biodistribution, cross the blood-brain barrier, do not bind to cell-surface proteins and are sufficiently stable.

In an example called ChemoTrace®, crucial time is saved during cancer treatment. ChemoTrace® instantly provides relevant data on the efficacy of the administered chemotherapeutic drug. An advantage of the present system is that it informs within 24-36 hours after a start of chemotherapy whether or not the therapy is effective. The use of ChemoTrace® will prevent patients from enduring heavy treatments without clinical benefits, which is regarded a major improvement in cancer therapy. The use of ChemoTrace® will result in cost savings.

It is noted that unfortunately a (positive) response rate to chemotherapy is limited to 20-35 %. Further, the number of treatment cycles is typically limited to a maximum of four due to limitations of the human body. In other words it is crucial to identify a suitable therapy right from the start, before starting a cycle. By identifying at an early stage of treatment if a treatment is effective the treatment per se can be carried out if considered effective and can be skipped if considered not effective. In the case of a not effective treatment a second treatment can be started likewise. Such can be repeated a further number of times. Once a treatment is considered effective a cycle of chemotherapy can be initiated. It is noted that the identification can be repeated for every and any chemotherapy.

ChemoTrace® prevents over-treatment, saving on medicines used to prevent side-effects of cytotoxic drugs.

With the present system there is no need to culture harvested tumour cell e.g. in order to perform tests, which can take a relatively long period of time e.g. a week. The necrotic cells are marked in their natural environment, which e.g. reduces a risk of human errors.

ChemoTrace® can be used to measure cytotoxic drug efficiency *in vivo* for all solid tumours which makes it an ideal system for a clinic. An advantage thereof is that in vivo relates to actual characteristics whereas *in vitro* may introduce deviations from the actual characteristics.

ChemoTrace® provides real-time data in the above respect, without a need to perform a puncture to harvest tumour cells. In this respect it is noted that by puncturing only a sample of a tumour is obtained. The obtained sample is not necessarily a true representation of the tumour. Further it is noted that a tumour is morphologically heterogeneous. Even further, the tumour may develop in morphological characteristics over time. The consequence hereof is that a punctured sample at the most gives an indication of characteristics of the tumour. The present invention does give the characteristics in real-time and with a high degree of certainty.

ChemoTrace® provides solid conclusions on the above points, such as through direct measurements, whereas prior art techniques only provide an indication of characteristics.

In an aspect, the present invention describes a device which measures the effect on tumour cells as well on healthy tissue caused by radiotherapy, called Actinotrace®.

In an aspect, the present invention describes a device which is a total body scan for detecting dead cells in the human body for prevention purposes, called NC-Scan®

In an aspect, the present invention describes a delivery platform for treatment of cancer by targeting of necrotic cells in a tumour, called Ilumicore®.

In an aspect, the present invention describes a platform to deliver active and regenerative compounds to a necrotic area caused by an ischemic event like a myocardial infarct or stroke, liver, kidney or other organ ischema, trauma, arthrosis or burn wounds, called Regenext®.

In an aspect, the invention describes a non-nuclear alternative for a mammograph scanner, called Mammolight®.

In an aspect, the present invention describes a device for targeted delivery of (chemo)therapeutics in solid tumours, called Chemoclick®.

In an aspect, the invention describes a targeted thermal therapy (Hyperthermic therapy), called Hyperthermys®.

Present diseases relate, amongst others to cancer, infarcted tissue of e.g. heart and brain, osteoarthritis, neurological diseases such as Alzheimer's disease.

The present invention also describes_use of the composition as a medicament; for use of the system in a diagnostic method, use of the system for treatment of diseases involving necrotic cell death, to use of the composition for detecting and/or targeting necrotic cells in vivo and/or in vitro; to a dosage comprising said system; to a method for determining localization of the composition within a sample using said composition; and, to a method for treatment of cancers and/or diseases involving necrotic cell death using said composition.

Targeting molecules of the present invention have been found to bind to dead (necrotic) cells very selectively.

As mentioned earlier it has been found by the present inventors that necrotic cells and/or necrosis in general are attractive targets. Such relates to the observation that regions of necrotic cells are typically present in cancers (tumours), e.g. due to an insufficient blood supply and thus lack of oxygen, and in (or result from) diseases involving necrotic cell death. It is noted that regions of necrotic cells are not typically found inside of healthy tissue. Examples of diseases involving necrotic cell death include infarcts or ischemic injury (i.e. Myocardial infarct, stroke, kidney, liver etc), trauma (i.e. brain, muscle, bone) infections or inflammation (i.e. septic shock, rheumatoid arthritis, osteonecrosis), degenerative diseases (i.e. Alzheimer, Parkinson dementia), plaques (arteriosclerotic, amyloid) burn wounds, irradiation induced necrosis and diabetes.

With regards to cancer, once a tumour has been identified, further necrotic cell death can be induced intentionally (for instance by local irradiation, photo dynamic or local thermal therapy and/or focused ultrasound) in part of the tumour to provide a larger target for the composition. Furthermore, wherein the present composition is used for therapeutic purposes, the number of necrotic cells will increase as the therapy progresses thus resulting in dose amplification as a function of time.

A useful discussion on the classification of cell death can be found in Kroemer et al, Cell Death and Differentiation, 2005, 12, 1463*.* In the present application, necrotic cells are taken as cells whose plasma membrane has lost integrity. A person of skill in the art is able to determine whether a plasma membrane is intact i.e. integral, such as through using fluorescent dyes, such as using commercial amine reactive dyes. The selectivity of the composition of the present invention for dead cells, i.e. cells whose plasma membrane has lost integrity, can be demonstrated in *in vitro* tests as will be shown in the examples below. It is important to note that amine reactive dyes which bind to dead cells are useful only *in vitro*. *In vivo*, they would react with amines e.g. of proteins or other compounds in blood, and would not be able to serve to target necrotic cells.

The term selectively indicates that the targeting compound has a higher affinity for necrotic cells than for healthy cells (thus the targeting molecules may target necrotic cells). Such can be determined in an *in vitro* assay as per the examples herewith, or e.g. by flow cytometry; in both methods, co-staining may be used e.g. using commercial live-dead cell staining kits. In simple terms, selective binding in the present invention indicates that for a given population of cells comprising necrosis and healthy cells the number of targeting molecules bound to necrotic cells is at least one order of magnitude higher than the number of targeting molecules bound to healthy cells, typically a few orders of magnitude, and preferably 6 or more orders of magnitude, such as 9 orders.

Non-activated cyanines are cyanines that are non-reactive towards e.g. amines and thiols. Non-activated cyanines cannot significantly (reaction is thermodynamically unfavourable) bind to dead cells (functional groups of molecules thereof) through covalently attaching to amines, thiols or other reactive functional groups present on molecules found inside of cells. That is to say that selective binding to necrotic cells in the context of the present invention is not through covalent bonding, but rather through non-covalent binding via the cynanine core structure and not through the side chains to which the active groups are attached. The term activated cyanine is known to a person of skill in the art and includes e.g. carboxylic acids activated as esters, N-hydroxysuccinimide esters, maleimides, acyl chlorides, SDS esters, etc. Non-activated cyanines include e.g. cyaninines comprising carboxylic acid functions i.e. the carboxylic acid is not activated.

It is noted that activated cyanines may be used in the formation of the composition e.g. for the purpose of attaching an imaging and/or therapeutic compound to the cyanine; in the composition however, the cyanine is non-activated and will still bind to necrotic cells through the cyanine core structure.

Advantages of the present description are detailed throughout the description.

### DETAILED DESCRIPTION

It is noted that examples given, as well as embodiments are not considered to be limiting. The scope of the invention is defined by the claims.

In a first aspect the present invention describes a system comprising a targeting molecule for binding necrotic cells, the targeting molecule being selected from a cyanine or any other fluorescent dye, including Rhodamines, that specifically non-covalently binds to intracellular proteins. Proteins for binding may relate to fibrous proteins, such as 40 kDa proteins, 100 kDa proteins, such as tubulin, such as α-tubulin, β-tubulin, γ-tubulin, δ-tubulin and ε-tubulin, actin, such as G-actin, and F-actin, fibrous structural proteins, such as keratin, such as neutral, basic or acidic keratin, such as keratin 1 - keratin 20, metalloenzymes, such as enolase, and lyase, CDC37, preferably tubulin, most preferably actin, isomers thereof, complexes thereof, and decay products thereof. These proteins are only available for the targeting molecule when the membrane integrity is lost, i.e. in case of a necrotic cell. Selectivity for binding nectrotic cells can be determined e.g. using the dry-ice dead-cell assay described below, in combination with fluorescent microscopy. Preferably there is least a 2-times higher affinity for necrotic cells compared to live cells, preferably at least 10-times, most preferably at least 1000-times, such as at least 1000000-times. Similarly, the affinity of the targeting molecule for one or more of the above proteins compared to an affinity for DNA is at least 2-times higher, preferably at least 10-times higher, most preferably at least 1000-times higher, such as at least 1000000-times higher, such as the targeting molecule does not and/or cannot bind to DNA i.e. the targeting molecule does not significantly bind to DNA or RNA.

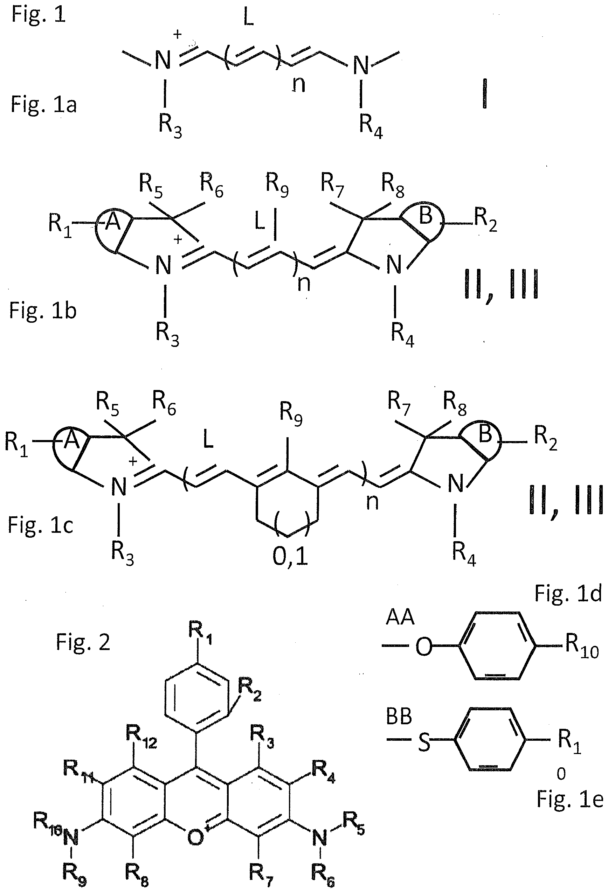

The targeting molecule is a non-reactive cyanine dye, according to Figure 1 I, II and III, wherein n is an integer, such as n ∈ [2,10], preferably n ∈ [4,8], the chain L has up to n-1 double bonds, preferably n/2 double bonds,
wherein sub-families II and III may comprise respectively one and two aromatic ring systems (A,B) signified by the curved line(s) C,
wherein A,B are preferably selected each individually from benzene and naphthalene, wherein further groups R₅, R₆, R₇, and R₈, may be present, R₅, R₆, R₇, and R₈, are preferably selected each individually from H, and alkyl, such as methyl, ethyl, and propyl, preferably methyl,
wherein the aromatic ring systems may comprise further functional groups R₁, R₂, and/or substituents, R₁, R₂, are preferably selected each individually from H, sulphonate, and sulphonamide,
wherein the chain of alternating single and double bonds L may be interrupted by one or more partly and fully saturated ring structures, such as cyclopentene and cylcohexene, and combinations thereof, such as one or more cyclohexene rings, wherein the saturated ring structure may further comprise functional groups Rg, being selected from H, AA and BB, wherein R₁₀ is selected from, H, SO₃H, Cl, -N-C=O-(CH2)_{q}-Y₃ (q=1-6), -(CH2)ᵣ-Y₄ (r=1-6), Y₃ and Y₄ are each independently one of H, COOH, SO₃H, and CN,
wherein the nitrogen atoms (N) may comprise further functional N-side groups R₃, R₄, wherein R₃, R₄ are preferably selected each individually from -(CH₂)ₘY, wherein Y is selected each individually from a carboxylic acid having 1-4 carbon atoms, a sulphonate group, CN, C≡C, and C=C, and salts thereof,
wherein said N-side groups comprise m carbon atoms, such as m ∈ [1,10], preferably m ∈ [2,8], more preferably m ∈ [3,7], most preferably m= 4,5, and 6, even more preferably at least one of m = 4, 5, and 6, preferably one m = 6, and the other m preferably is 4, 5 or 6,
wherein said N-side groups comprise one or more functional groups on an end opposing the N, such as a carboxylic acid having 1-4 carbon atoms, an sulphonic group, and salts thereof, such as sodium and potassium salts, most preferably the functional group on the end comprises one or more double C-C bonds, preferably a carboxylate thereof, and/or wherein the targeting molecule is neutral or negatively charged. Upon testing especially the above cyanines have been found to be very suitable, e.g. in terms of selectivity. Advantageously, cyanines of the invention having a negative charge have been found to bind preferentially to intracellular proteins in the presence of other cell components such as e.g. DNA and RNA. That is to say, cyanines of the invention having a negative charge show in general no significant binding to DNA or RNA.

The targeting molecule is preferably selected from: HQ4, HQ5, HQ6, HQ7, ICG, CW 800, ZW800, L4, L7, L11, CY3, CY3b, CY3.5, CY5, CY5.5, CY7, Dy 676, Dy 681, Dy 731, Dy 751 and Dy 776; and, is most preferably selected from HQ4, HQ5, CW800 and ZW800. These compounds are presented in Figure 2. Some of these compounds have for instance very good fluorescent properties. Such is rather unexpected, as a compound per se might have good fluorescent properties, but such may change upon binding to a further molecule, such as the present protein.

In an example, the agent is (one or more of the following (a), (b) and (c)): (a) an imaging compound selected from a group consisting a luminescent compound, a radioactive tracer, an MRI contrast or Spectroscopic agent, a RFA(radiofrequency ablation) agent, a PET agent, SPECT agent, ultrasound microbubble or contrast agent, opto-acoustic nanoparticles or contrast agent, CT contrast agent, a Raman agent or combinations thereof. In other words a suitable imaging compound may be used with the present system; surprisingly a wide range of such imaging compounds may be attached to the present system. As such a wide range of imaging techniques have become available. Such imaging techniques have been found to be very sensitive, i.e. small amounts of necrotic cells may be detected, very accurate, i.e. very small locations comprising necrotic cells may be identified, very reliable, and very confident, e.g. in terms of results. The agent may also be a therapeutic compound. As above the therapeutic compound can be brought to necrotic cells, using a small amount thereof, and yet providing a relative high amount thereof at an intended location, i.e. necrotic cells. The therapeutic compound (or one or more thereof) may be selected from a group consisting of chemo therapeutic agents, photo dynamic compounds, hyperthermic compounds (including photo thermal agents), immune modulating agents, proteins and peptides, nucleic acids (RNA- or DNA-based), medicaments (like antibodies), and/or combinations thereof. As such many therapies can be carried out, as well as combinations thereof. The agent may also be one or more release factors for stimulating tissue regeneration, such as growth factors and stem cells. A such repair and/or regeneration of tissues and cells are possible.

In an example, the vehicle is selected from a group consisting: a liposome, a dendrimer, a biodegradable particle, a micelle, a nanoparticle, an acoustic dissociable particle, a pH dissociable particle, a light dissociable particle, a heat dissociable particle, a chelating moiety and combinations thereof. Depending on the present system, e.g. in terms of further components being present, a suitable vehicle may be selected. Likewise a combination of vehicles may be selected. For instance a chelating moiety may be used for transporting In, F, Ga, Gd and Tc.

Vehicles may be considered substances that serve as mechanisms to improve the delivery and the effectiveness of drugs. Vehicles may be used for controlled-release in order to decrease drug metabolism, in order to prolong in vivo drug actions, and in order to reduce drug toxicity. Present vehicles are also used to increase effectiveness of drug delivery to a target site, e.g. of pharmacological action.

In an example, the in the body non-reactive molecule, which may be referred to as click A, is being capable of interacting with a counterpart, which may be referred to as click B. The non-reactive molecule is preferably one or more of a first Click-Chemistry component, a second Click-Chemistry component (complementary to the first Click-Chemistry component), and a component of a self-labelling protein tag such as of Snap-Tag. Preferably the Click-Chemistry components 'click' without requiring a catalyst such as copper (I) .

In a second aspect the present invention describes the system of the invention for use as a medicament. As mentioned above the present system may comprise a therapeutic compound. The system may therefor act as a medicament. It is preferred to use the present medicament for treating diseases, illnesses, etc. as identified throughout the application.

In a third aspect the present invention describes use of the system of the invention in a diagnostic method, such as MRI, SPECT, RFA (Radiofrequency ablation), PET, CT, Raman spectroscopy, Ultrasound, Optical and Opto-acoustic. As the present system has superior characteristics for imaging, diagnosis by one or more of the aforementioned methods is preferred.

In a fourth aspect the present invention describes the system of the invention for use in treatment of diseases involving necrotic cell death, including infarcts or ischemic injury, such as Myocardial infarct, stroke, and kidney, trauma, such as in a brain, a muscle, a bone, infections or inflammation, such as septic shock, rheumatoid arthritis, and osteonecrosis, degenerative diseases, such as Alzheimer, Parkinson dementia, plaques, such as arteriosclerotic, and amyloid, and Diabetes. Further examples of diseases for which the system may be used for treatment are included throughout the description.

In an aspect the present invention relates to a dosage for detection and/or treatment of cancers and/or diseases involving necrotic cell death comprising an effective amount of the system of the invention.

The dosage comprises an amount of 0.1-1000 nMole system/kg body weight, preferably 0.5-500 nMole system/kg body weight, more preferably 1-250 nMole system/kg body weight, even more preferably 2-100 nMole system/kg body weight, such as 5-50 nMole system/kg body weight; such may relate to a dosage of e.g. 0.01-1 mgram. The dosage preferably is provided in a physiological solution of 1-50 ml. Preferable a kit comprising some (1-50)dosages is provided.

In a sixth aspect the present invention describes a method for determining localization within a sample, said sample comprising a population of cells comprising necrotic cells, comprising:
(a) providing a system according to the present invention;
(b) performing one or more measurements on the sample with at least a first suitable imaging technique providing a value or values, and;
(c) analysing said value or values of the measurement to determine localization, optionally by comparison with a set of reference values. At least some of the above mentioned techniques are very suitable in this respect. The method may be carried out in vivo and in vitro.

In a seventh aspect the present invention describes a method for treatment of cancers and/or diseases involving necrosis comprising:
(a) administering the composition of the present invention wherein the composition comprises one or more therapeutic compound(s)
(b) determining localization of the composition within the cancer and/or necrotic cell death
(c) activating release of at least the therapeutic compound(s) from the vehicle and/or activating the therapeutic compound(s). The treatment may be carried out in vivo.

The invention is further detailed by the Examples and accompanying figures, which are exemplary and explanatory of nature and are not limiting the scope of the invention. To the person skilled in the art it may be clear that many variants, being obvious or not, may be conceivable falling within the scope of protection, defined by the present claims.

### FIGURES

**Figs. 1a****-c** show generic structures of three main sub-families of the present cyanine. Cyanine is a non-systematic name of a synthetic dye family belonging to polymethine group. Referring to the central carbon chain in figure 1; n is an integer, such as n ∈ [2,10], preferably n ∈ [4,8]. The chain L has up to n-1 double bonds, preferably n/2 double bonds. Sub-families II and III may comprise respectively one and two aromatic ring systems (A,B) signified by the curved line(s) C. A,B are preferably selected each individually from benzene and naphthalene. Groups R₅, R₆, R₇, and R₈, may be present. R₅, R₆, R₇, and R₈, are preferably selected each individually from H, and alkyl, such as methyl, ethyl, and propyl, preferably methyl. The aromatic ring systems may comprise further functional groups R₁, R₂, and/or substituents. R₁, R₂, are preferably selected each individually from H, sulphonate, and sulphonamide. The chain of alternating single and double bonds L may be interrupted by one or more partly and fully saturated ring structures, such as cyclopentene and cylcohexene, and combinations thereof, such as one or more cyclohexene rings. The saturated ring structure may further comprise functional groups R₉, R₉ being selected from H, AA and BB. R₁₀ is selected from, H, SO₃H, Cl, -N-C=O-(CH2)_{q}-Y₃ (q=1-6), -(CH2)ᵣ-Y₄ (r=1-6). Y₃ and Y₄ are each independently one of H, COOH, SO₃H, CN. The nitrogen atoms (N) may comprise further functional N-side groups R₃, R₄. R₃, R₄ are preferably selected each individually from - (CH₂)ₘY. Y is selected each individually from a carboxylic acid having 1-4 carbon atoms, a sulphonate group, CN, C=C, and C=C, and salts thereof. N-side groups comprise m carbon atoms, such as m ∈ [1,10], preferably m ∈ [2,8], more preferably m ∈ [3,7], most preferably m= 4,5, and 6, even more preferably at least one of m = 4, 5, and 6, preferably one m = 6, and the other m preferably is 4, 5 or 6. The N-side groups comprise one or more functional groups on an end opposing the N, such as a carboxylic acid having 1-4 carbon atoms, an sulphonic group, and salts thereof, such as sodium and potassium salts. Most preferably the functional group on the end comprises one or more double C-C bonds.
   The term cyanine refers to any compound whose core-structure is that of sub-family I, II or III. The integer in names of cyanines such as Cy 3, Cy 5, Cy 7 etc. refers to the number of carbon atoms in the chain L. In an exemplary embodiment, the cyanine belongs to one of these families.
**Fig. 2** shows a generic structure of a Rhodamine; R1 to R12 can be hydrogen or a functional group, examples of suitable functional groups include sulphonic acid groups, carboxylic acid groups, sulphonamides, alcohols, amines, esters, ethers, thiols, thio esters and combinations thereof. The term Rhodamine refers to any compound whose core-structure is that shown in Fig 2.

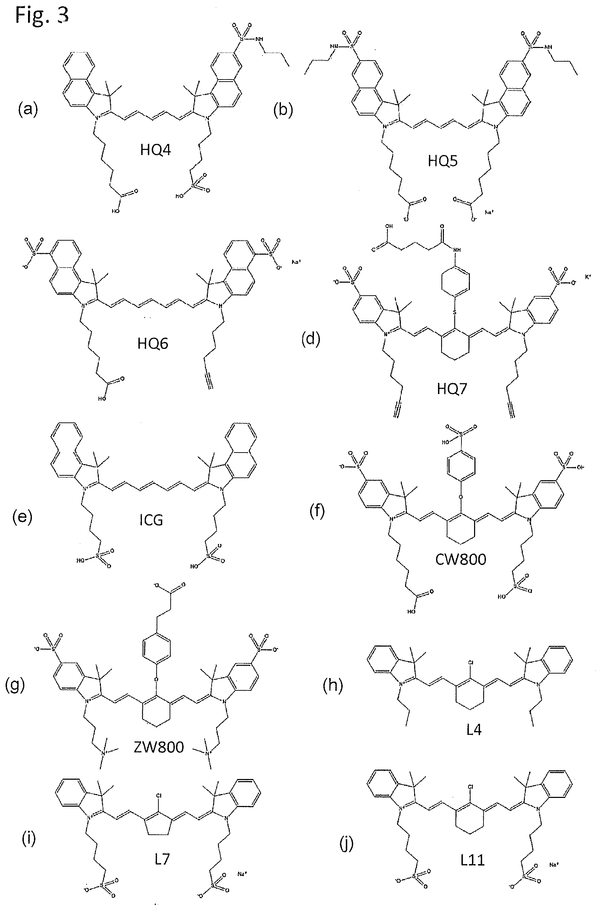

**Fig. 3** (a)-(j) gives the structures of compounds referred to throughout the application. Where a counterion is shown, this may be for example H⁺, Na⁺ or K⁺.

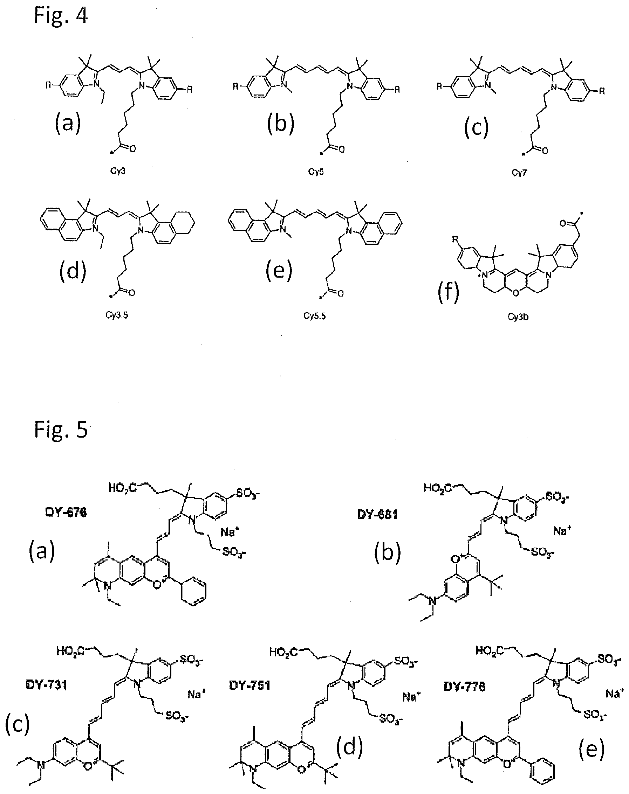

**Fig. 4-5** give preferred examples of the present targeting molecules.

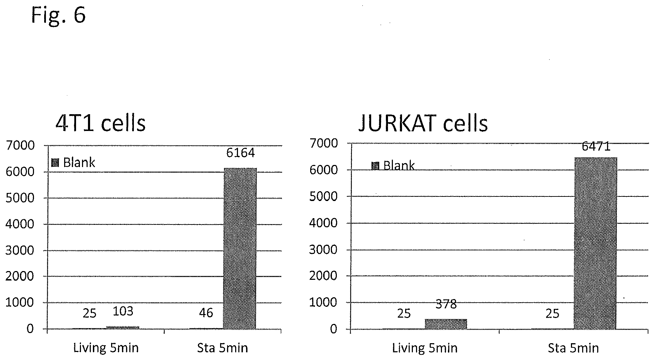

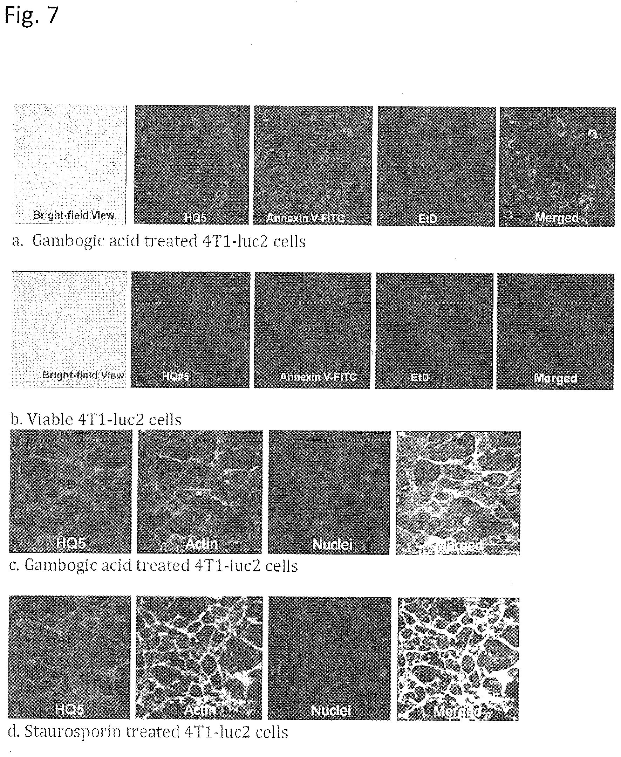

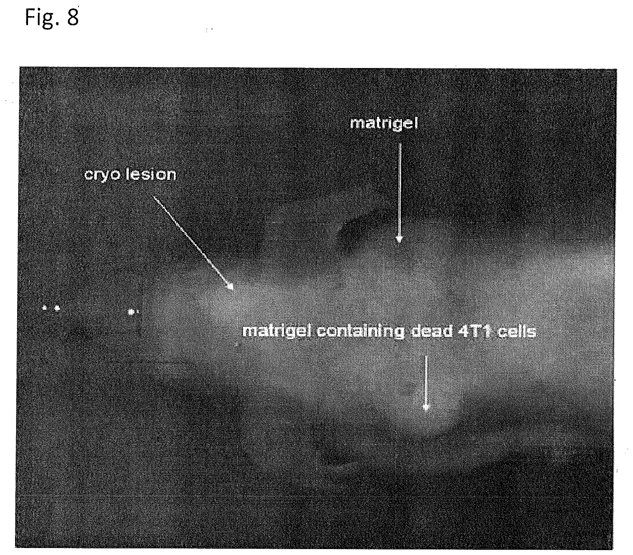

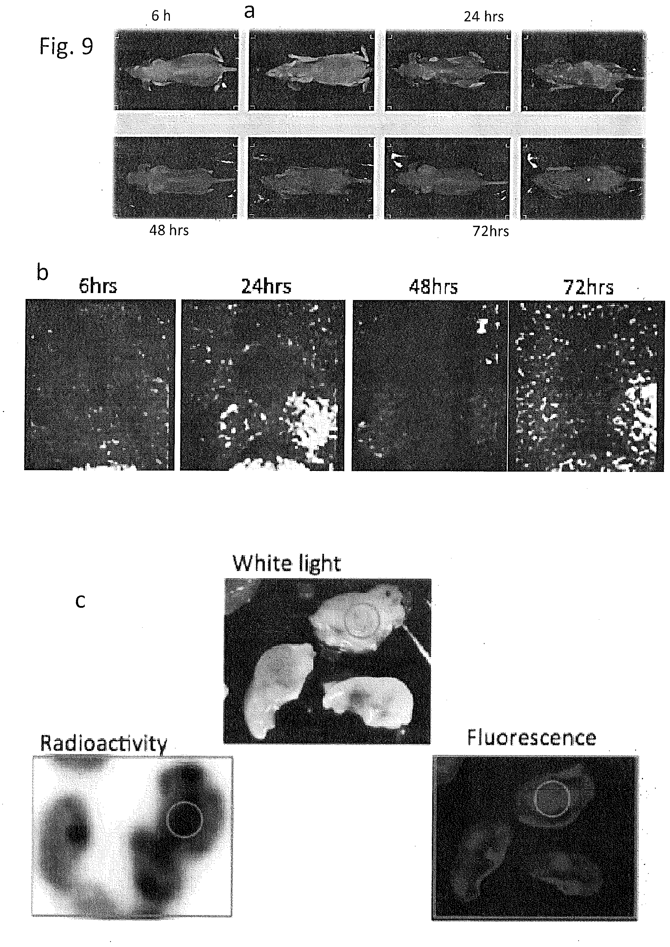

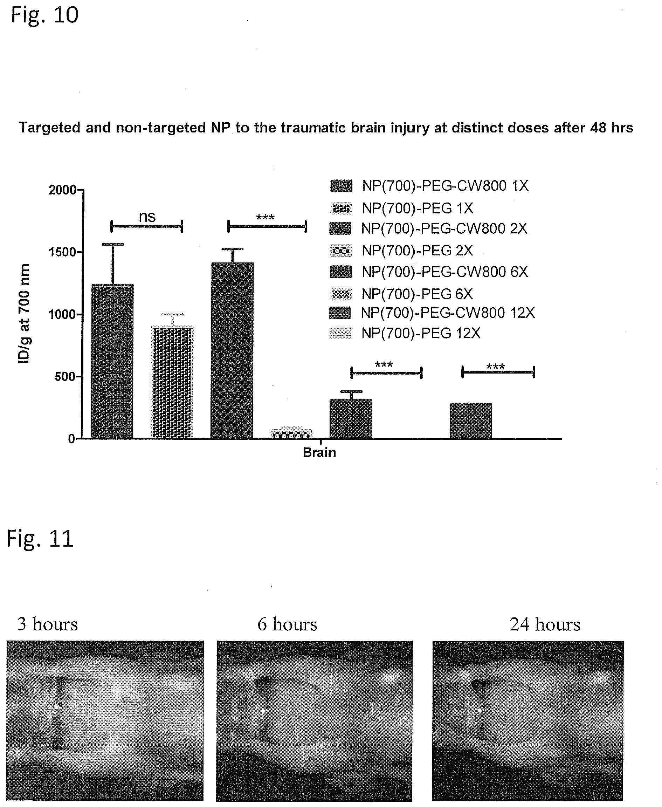

**Fig. 6-11** show results of experiments; these are detailed below.
**Fig. 12** shows a schematic representation of NP-CW800.
**Fig. 13** shows structure of DTPA-CO-NH-PEG-NH₂.
**Fig. 14** shows structure of DTPA-CO-NH-PEG-HQ4.
**Fig. 15a,b** shows structures of two variants of Click A-HQ4-DTPA.
**Fig. 16** shows structure of Click A-ZW800-DTPA.
**Fig. 17a,b** show structures of two variants of Click A-ZW800-NOTA.
**Fig. 18** shows structure of ClickA-ZW800 modified (at ammonium groups)-NOTA.
**Fig. 19a** shows structure of ZW800-linker-NOTA and Click A
**Fig. 19b** shows structure of HQ4-linker-NOTA and Click A With reference to Figures 6-11:

### Measurement technology

In this investigation several existing and/or in-house developed techniques have been applied:

### Fluorescence

[Wikipedia:] "Fluorescence is the emission of light by a substance that has absorbed light or other electromagnetic radiation of a certain wavelength. In most cases, the emitted light has a longer wavelength, and therefore lower energy, than the absorbed radiation."

Measurements with fluorescence, so also in-vivo fluorescence, are hard to quantify. The measured intensity in a living creature or cell tissue is -besides the intrinsic brightness of the fluorescent compound itself and its concentration- also dependent of the physical properties of the chemical compound, i.e. the binding ratio with its surrounding proteins and the kind of interactions with them and the distribution of the substance through the tissue.

Finally, the sensitivity and specificity of the measurements are very strongly dependent of the experiment protocol.

### Dry-ice dead cell-assay

Solid carbon dioxide in ethanol (-80 °C) is used to freeze the middle of a well of a 24 well-plate with a culture of living cells (and possibly other components). The procedure has been optimized by using a cooled block with pins that is held for a certain period of time (mostly approx. 15 sec.) to the bottom of the 24-well plate. The result is the freezing and consequent death of the cells in the middle surrounded by still living cells, enabling comparative studies between dead and living cells.

A medium containing compounds that can discriminate between living and dead cells is applied to the culture wells. After incubation the cell layer is washed and imaged.

### Cryolesion mouse model

A small metal cylinder of 3 mm diameter is precooled in liquid nitrogen and applied to the parietal region of an anesthetized mouse's head for either 20 or 60 sec. After a certain period of time the dye is injected (in a certain concentration) in the tail vein. 3 - 24 hours after injection the mice can be imaged.

The selectivity of the composition of the present invention for necrotic cells, i.e. cells whose plasma membrane has lost integrity, is demonstrated in *in vitro* tests as will be shown in the examples below.

### Matrigel

Matrigel is a gelatinous protein mixture secreted by specific mouse sarcoma cells. In this invention it is used as an endogenous matrix for dead cells that is implanted under the skin of a mouse. If these cells are also labelled it is confirmed that the dyes and constructs are distributed over the whole body, so also outside the bloodstream.

### MSOT

Multispectral Optoacoustic Tomography is a technique for whole-body imaging of bio chemical markers in small animals. The absorption of light causes a small thermal expansion that generates acoustic waves. These can be detected and converted in a three-dimensional image.

### PROOF OF CONCEPT

### Figure 6

Flow cytometry data using HQ5 on adherent (4T1) cells and non-adherent (JURKAT) cells treated or not treated with the cytotoxic agent Staurosporin. The probe only stains staurosporin treated cells, which have lost membrane integrity.

### Figure 7

Confocal microscopy of: a: gambogic acid (a necrosis inducing agent) treated cells and b: viable cells. C-D: Co-staining of a F-actin using phalloidin co-localized with HQ5 indicating actin binding of HQ5. (c: Gambogic acid treated cells and d: Staurosporin treated cells).

### Figure 8

To show the capacity of HQ5 to bind necrotic cells in vivo a local cryolesion of the brain was induced prior to HQ5 injection. In addition matrigel with or without necrotic cells was transplanted subcutaneously. HQ5 accumulates specifically in both the cryo-lesion and matrigel with necrotic cells, but not in the matrigel without necrotic cells.

### Figure 9

To show the capacity of HQ5 to bind necrotic cells in vivo in a more physiological model than cryolesions, HQ 4-DTPA containing radioactive In-111 was injected in mice bearing tumours with a necrotic core. A. Tumour specific HQ4 fluorescence was observed 24 hours after probe injection, lasting at least to 72 hours. B. A specific radioactive SPECT signal was detected 24 hours after probe injection, lasting at least to 72 hours. C. Ex vivo colocalization of both the fluorescent signal and radioactive signal with the necrotic core was observed.

### Figure 10

In order to investigate the possibility of using cyanine dyes to deliver PLGA nanoparticles to necrotic areas, PLGA nanoparticles coated with or without CW800 were injected into animals with a cryolesion of the brain. At low concentrations (diluted 2 times or more) CW800 coated PLGA nanoparticles accumulated selectively in the necrotic area whereas the non-coated control PLGA nanoparticles did not.

### Figure 11

Photodynamic therapy is known to cause necrosis. CW800 coated PLGA nanoparticles were injected 6 hours after photodynamic treatment of a 4T1 tumour on the left flank of the animal. A second tumour on the right flank remained untreated. The CW800 coated PLGA nanoparticles only accumulated at the site of the treated tumour.

### Examplary systems of the invention

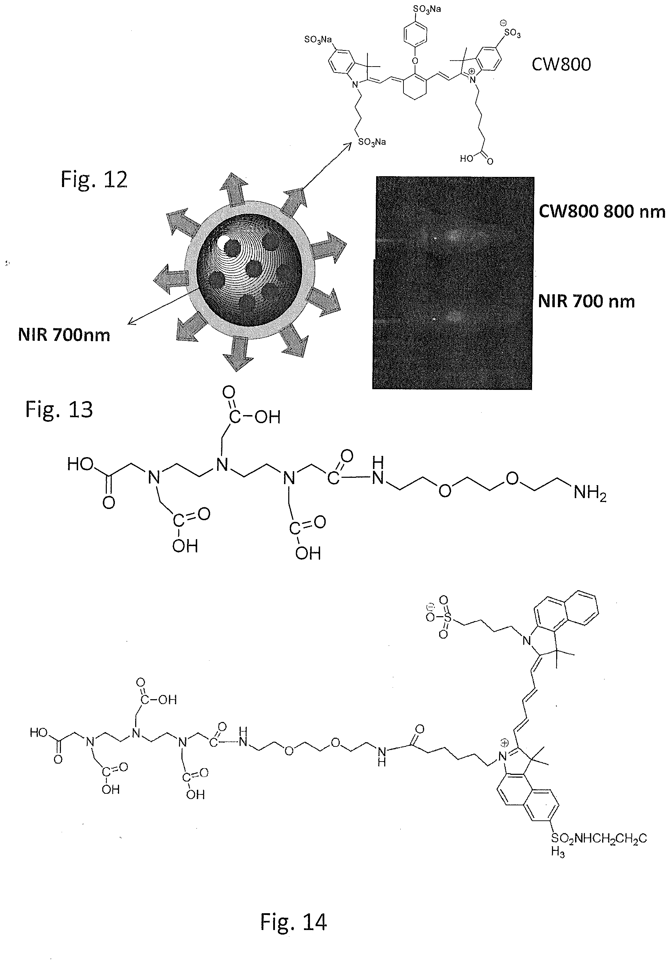

### Example 1 - With reference to Figure 12

**PLGA NP preparation**. PLGA NPs with entrapped near-infrared fluorescently labeled was prepared using an o/w emulsion and solvent evaporation-extraction method. In brief, 90 mg of PLGA in 3 mL of DCM containing the near-infrared (NIR) 700 nm (680R from LI-COR) (3 mg) was added drop-wise to 25 mL of aqueous 2% (w/v) PVA in distilled water and emulsified for 90 seconds using a sonicator (Branson, sonofier 250). A combination of lipids (DSPE-PEG(2000) amine (8 mg) and mPEG 2000 PE (8 mg)) were dissolved in DCM and added to the vial. The DCM was removed by a stream of nitrogen gas. Subsequently, the emulsion was rapidly added to the vial containing the lipids and the solution was homogenized during 30 seconds using a sonicator. Following overnight evaporation of the solvent at 4°C, the PLGA NPs were collected by ultracentrifugation at 60000 g for 30 min, washed three times with distilled water and lyophilized.

**Conjugating CW800-NHS to the PLGA NPs.** CW800-NHS were conjugated to the DSPE-PEG(2000)-amine-containing PLGA NPs preparation. Subsequently, particles (50 mg) were dissolved in 0.5 mL of carbonated buffer (pH 8.5) and the activated CW800-NHS (1 mg) was added to the particles during 1 h at room temperature. Unbound CW800-NHS was removed by centrifugation (10000 g, during 10 min) and the PLGA NPs-CW800 was washed four times with PBS. The amount of CW800 on the particle surface was determined by Odyssey scanning (table 1).

**Dynamic Light Scattering and Zeta-Potential**. Dynamic light scattering (DLS) measurements on all nanoparticles were performed on an ALV light-scattering instrument equipped with an ALV5000/60X0 Multiple Tau Correlator and an Oxxius SLIM-532 150 mW DPSS laser operated at a wavelength of 532 nm. A refractive index matching bath of filtered cis-decalin surrounded the cylindrical scattering cell, and the temperature was controlled at 21.5 ± 0.3°C using a Haake F3-K thermostat. For each sample the auto-correlation function, g₂(τ), was recorded ten times at a detection angle of 90°. For each measurement the diffusion coefficient, D, was determined using the 2^{nd} order cumulant and the corresponding particle diameter was calculated assuming that the particles were spherical in shape (see table 1). Zeta potential of all nanoparticles was determined on a Malvern ZetaSizer 2000 (UK).

| NPs with and without targeting moiety | Nanosphere size ± S.D. (nm) | PDI | Width (nm) | Zeta potential ± S.D. (mV) | Targeting moiety CW800 ug/mg PLGA) (w/w) | Ligands (# ligands/NP) (Experimental values) |
|---|---|---|---|---|---|---|
| PLGA-NP (NIR-680)-PEG-Not | 214,0 ± 12.3 | 0,204 | 75.6 | -25.4 ± 1.60 | ---- | ---- |
| PLGA-NP (NIR-680)-PEG-CW800 | 216,2 ± 10.5 | 0.196 | 73.2 | -41.5 ± 3.25 | 0.25 ug | 866 |

### Example 2 - with reference to Figures 13

### Synthesis of DTPA-PEG-NH2.

DTPA containing PEG amine link (4,7,10-Trioxa-1,13-tridecanediamine, indicated as PEG-NH₂ in the formula (DTPA-PEG-NH2) was synthesised on Cl-TrtCl resin. Thus, Fmoc-PEG amine was incorporated on Cl-TrtCl resin (CTC resin) by reacting 3 eq. Fmoc-PEG amine in presence of 6 eq. DIEA in DCM during overnight at room temperature. Final loading was measured by Fmoc quantification: value obtained was around 0.8 mmol/g. Fmoc group removal was carried out with piperidine-DMF (1:5) (1 x 1 min, 2 x 10 min). Next, the DTPA-(tetra-tBu ester)-COOH (2 eq.) was coupled using DIPCDI (2 eq.) and HOBt (2 eq.) in DMF during overnight. After coupling overnight the ninhydrin test was negative. Later on, DTPA-(tetra-tBu ester)-CO-NH-PEG-CTC-resin cleavage and deprotection was performed in two steps. DTPA-(tetra-tBu ester)-CO-NH-PEG-CTC-resin was treated with 1% TFA in DCM for 10 times for 1 min each time. Excess DCM was removed using vacuum and side chain protecting groups were removed using 95% TFA, 2.5% TIS and 2.5% water. DTPA-CO-NH-PEG-NH2 was precipitated with cold MTBE after TFA removal under a N₂ stream. The DTPA-CO-NH-PEG-NH2 was dissolved in water and lyophilized to obtain the final product. The desired DTPA-CO-NH-PEG-NH2 was 85.0% in yield with a purity of 90.6% as analysed by HPLC (tR 2.28 min). HPLC-MS, m/z calc.: 523.25 for C₂₀H₃₇N₅O₁₁, Found: 524.5.28 [M+1]+ and MALDI-TOF Found 524.2 [M+1]+ 546.3 [M+Na].

### Example 3 - with reference to Figure 14

### Synthesis of DTPA-CO-NH-PEG-NH2 -HQ4.

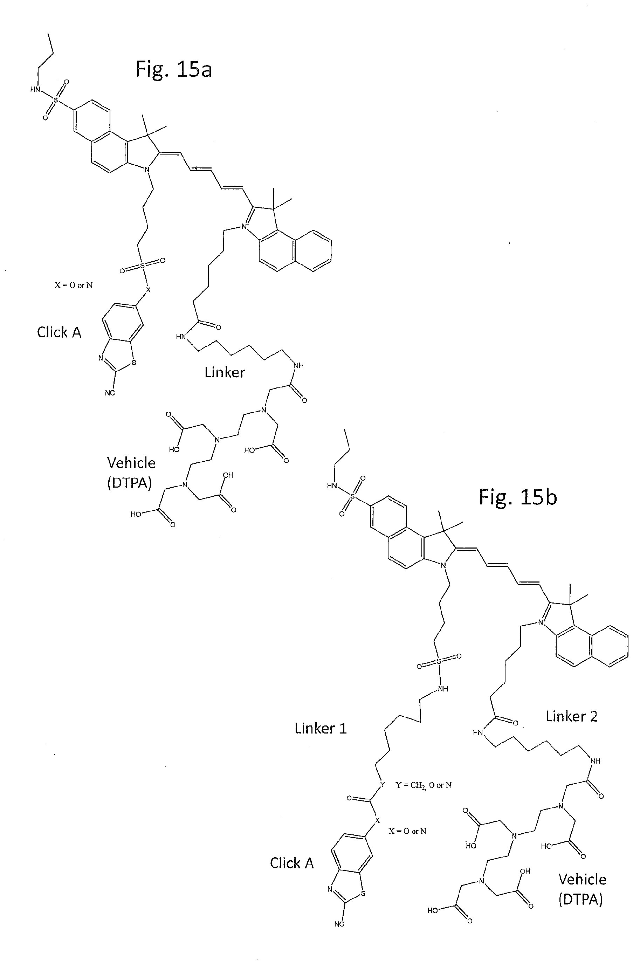

HQ4-NHS (8.3x10⁻⁴ mmol, 1 mg) dissolved in 50 µL of DMSO was added to DTPA-CO-NH-PEG-NH2 (2.8 x 10⁻³ mmol, 2 mg) dissolved in 200 µL of DMSO containing 5 µL DIEA and stirred overnight at room temperature. Later on, the complex DTPA-CO-NH-PEG-HQ4 was purified by RP-HPLC. The desired Dota-PEG-HQ5 was 50.5% in yield with a purity of 98% as analysed by HPLC (tR 5.34). HPLC-MS, m/z calc.: 1331.59 for C₆₆H₉₀N₈O₁₇S₂, Found: 1332.0[M+1]+ and MALDI-TOF Found 1331.9 [M+1]+ 1353.9 [M+Na]. Further Examples of systems according to the invention are shown in Figures 15-19.
Figure 15a and 15b - variants of Click A-HQ4-DTPA
Click A = 2-cyanobenzothiazole
Vehicle = Diethylenetriaminepentaacetic acid (DTPA)

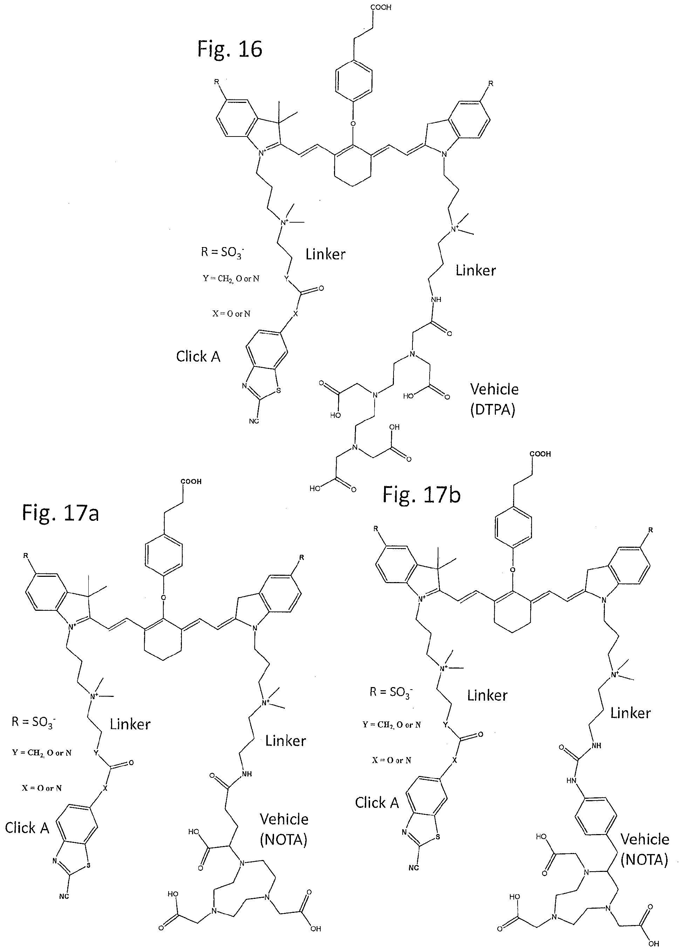

Figure 16 - ClickA-ZW800-DTPA
Click A = 2-cyanobenzothiazole
Vehicle = Diethylenetriaminepentaacetic acid (DTPA)
Figures 17a and 17b - variants of ClickA-ZW800-NOTA
Click A = 2-cyanobenzothiazole
Vehicle = 2,2,2-(1,4,7-triazanonane-1,4,7-triyl)-triacetate (NOTA)

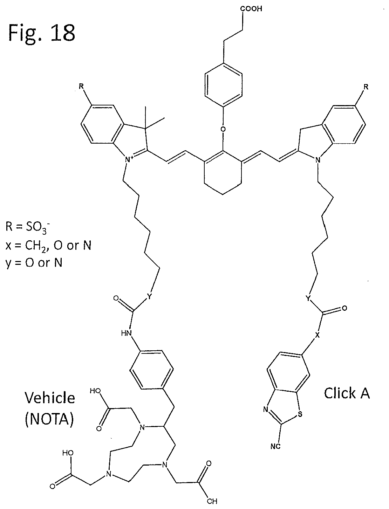

Figure 18 - ClickA-ZW800-modified (at ammonium groups)-NOTA
Click A = 2-cyanobenzothiazole
Vehicle = 2,2,2-(1,4,7-triazanonane-1,4,7-triyl)-triacetate (NOTA)

It is noted that whilst in examples 16-18, R=SO₃⁻ (i.e. the cyanine is compound ZW800), R may also be another moiety, such as e.g. H, alkyl etc, provided the resulting cyanine falls within the scope of one or more of the claims.

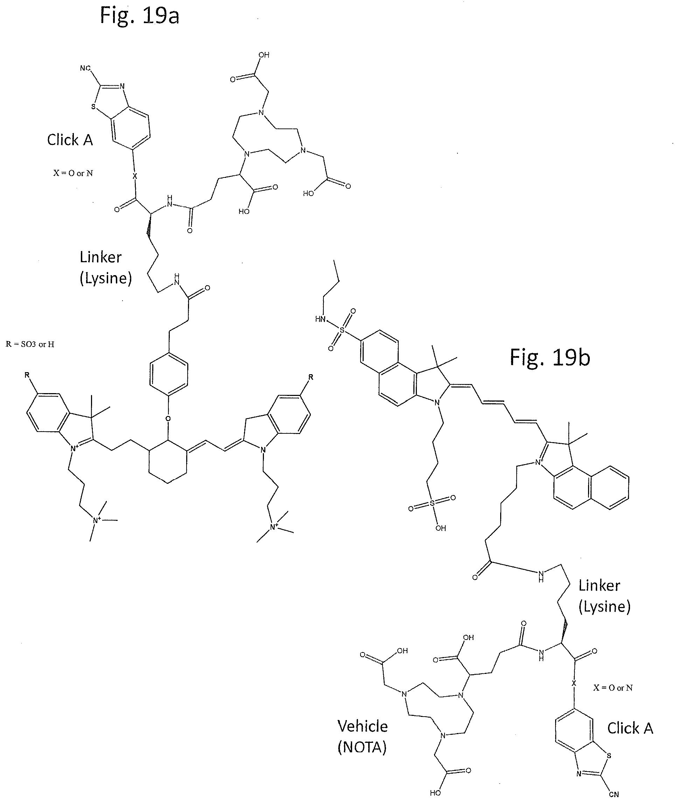

Figure 19a - ZW800-linker-NOTA and Click A
Click A = 2-cyanobenzothiazole
Vehicle = 2,2,2-(1,4,7-triazanonane-1,4,7-triyl)-triacetate (NOTA)
Figure 19b - HQ4-linker-NOTA and Click A
Click A = 2-cyanobenzothiazole
Vehicle = 2,2,2-(1,4,7-triazanonane-1,4,7-triyl)-triacetate (NOTA)

As an alternative to the systems of claims 15-18, wherein the vehicle and click A (in the body non-reactive molecule being capable of interacting with a counterpart)are provided on distinct first and second functional groups of the cyanine, both the vehicle and click A (in the body non-reactive molecule being capable of interacting with a counterpart) may be linked to a single functional group of the cyanine using a bifunctional linker, such as through lysine as shown in Figure 19. In figure 19a, click A and NOTA are linked through the carboxylic acid group of ZW800. A similar strategy may be applied to link via a side-arm i.e. the nitrogen of an ammonium group. A person skilled in the art will realise that other combinations of targeting molecule and: (a) agent, (b) vehicle and/or (c) in the body non-reactive molecule being capable of interacting with a counterpart, are possible e.g. Click A-HQ4-NOTA, Click A-HQ5-NOTA/DTPA, Click A-CW800-NOTA/DTPA, Click A-ZW800-NOTA/DTPA etc. NOTA and DTPA are examples of chelating ligands suitable for transporting an agent, such as a metal ion.

### Glossary

**4T1-cells** Breast cancer cells from murine origin.
**4T1-luc2** Same cells, but genetically modified to have lucifer-**ase-2**, an enzyme that can convert luciferine (luc) accompanied by the radiation of light. So, after administration of luc these cells can be detected.
**BSA** Bovine Serum Albumin, a water soluble protein from bovine serum used for all kinds of biological applications, among others to study the binding of a substance to proteins.
**CRO** Contract Research Organization
**FACS** Fluorescence Activated Cell Sorting, a technique where the fluorescence of a flow of individual cells can be measured.
**FCS** Fetal Calf Serum, a serum from mentioned origin, with comparable application as BSA Lysate A fluid containing the contents of cells that are broken down and where the integrity and organization is all gone.
**MALDI-TOF** Matrix-Assisted Laser Desorption/Ionization- Time-Of-Flight is an chemical analytical technique to determine the molecular mass of a chemical entity. MALDI is a 'soft' ionization technique, while TOF is a detection technology particularly suitable for large (heavy) molecules.
**MRI** Magnetic Resonance Imaging, a medical imaging technique, making use of the principle of NMR.
**MTS-assay** a calorimetric assay that measures the viability of the cell. The higher the signal, the more viable the cells are.
**MSOT** Multispectral Optoacoustic Tomography, see paragraph 3.2.
**NIRF** Near InfraRed Fluorescence
**NMR** Nuclear Magnetic Resonance, a physical phenomenon in which atoms with specific properties (i.e. being magnetic, having a spin) can absorb and re-emit radiation of an atom-specific wavelength.
**PDT** Photo dynamic therapy is a form of phototherapy using non-toxic light-sensitive com-pounds that are exposed selectively to light, whereupon they become toxic to targeted malignant and other diseased cells.
**PET** Positron Emission Tomography
**PKPD** PharmacoKinetics and PharmacoDynamics studies how the substance behaves in a living body (bioavailability, distribution, metabolism) and how it interacts with the biological target (binding, residence time)
**SDS-PAGE** Sodium Dodecyl Sulphate PolyacrylAmide Gel Electrophoresis is a technique to separate mixtures of proteins into individual proteins (or mixtures with less components)
**SPECT** Single-Photon Emission Computed Tomography is an imaging technique using gamma rays. It is able to provide true 3D information. This information is typically presented as cross-sectional slices through the patient, but can be freely reformatted or manipulated as required. [Wikipedia]
**TUNEL** Terminal deoxynucleotidyl transferase dUTP nick end labelling is a method for detecting DNA fragmentation by labelling the terminal end of nucleic acids [Wikipedia]. It is used as a labelling tool of necrotic tissue.

## Claims

1. Dosage for use in the detection and/or treatment of cancers and/or diseases involving necrotic cell death comprising an effective amount of a system comprising a first entity being a targeting molecule for binding necrotic cells, the targeting molecule being a cyanine that is non-activated, is capable of non-covalently binding to intracellular proteins when the membrane integrity of a cell is lost, and does not interact with DNA,
wherein the system further comprises a second entity, which the entities being joined, such as by a chemical bond, selected from one or more of:
(a) an agent selected from an imaging compound, a therapeutic compound, and a release factor;
(b) a vehicle that is effective for transporting an/the agent; and
(c) an in the body non-reactive molecule being capable of interacting with a counterpart,
where the targeting molecule is a non-reactive cyanine dye, according to figure 1 I, II and III, 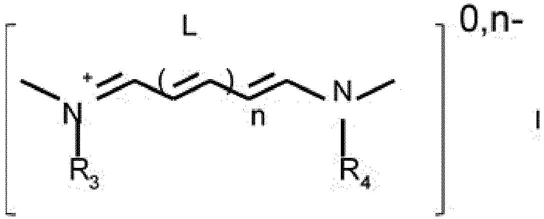 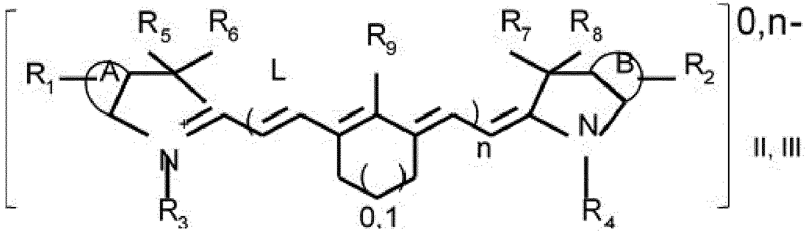 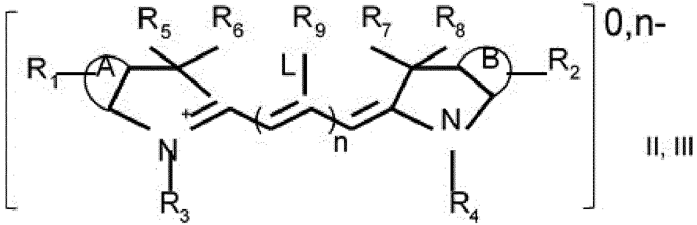
wherein n is an integer, such as n ∈ [2,10], preferably n ∈ [4,8], the chain L has up to n-1 double bonds, preferably n/2 double bonds,
wherein sub-families II and III may comprise respectively one and two aromatic ring systems (A,B) signified by the curved line(s) C,
wherein A,B are preferably selected each individually from benzene and naphthalene,
wherein further groups R₅, R₆, R₇, and R₈, may be present, R₅, R₆, R₇, and R₈, are preferably selected each individually from H, and alkyl, such as methyl, ethyl, and propyl, preferably methyl, wherein the aromatic ring systems may comprise further functional groups R₁, R₂, and/or substituents, R₁, R₂, are preferably selected each individually from H, sulphonate, and sulphonamide, wherein the chain of alternating single and double bonds L may be interrupted by one or more partly and fully saturated ring structures, such as cyclopentene and cylcohexene, and combinations thereof, such as one or more cyclohexene rings, wherein the saturated ring structure may further comprise functional groups Rg, being selected from H, AA and BB, wherein R₁₀ is selected from, H, SO₃H, Cl, -N-C=O-(CH2)_{q}-Y₃ (q=1-6), -(CH2)ᵣ-Y₄ (r=1-6), Y₃ and Y₄ are each independently one of H, COOH, SO₃H, and CN,
wherein the nitrogen atoms (N) may comprise further functional N-side groups R₃, R₄, wherein R₃, R₄ are preferably selected each individually from -(CH₂)ₘY, wherein Y is selected each individually from a carboxylic acid having 1-4 carbon atoms, a sulphonate group, CN, C=C, and C=C, and salts thereof,
wherein said N-side groups comprise m carbon atoms, such as m ∈ [1,10], preferably m ∈ [2,8], more preferably m ∈ [3,7], most preferably m= 4,5, and 6,
even more preferably at least one of m = 4, 5, and 6, preferably one m = 6, and the other m preferably is 4, 5 or 6,
wherein said N-side groups comprise one or more functional groups on an end opposing the N, such as a carboxylic acid having 1-4 carbon atoms, an sulphonic group, and salts thereof, such as sodium and potassium salts, most preferably the functional group on the end comprises one or more double C-C bonds, preferably a carboxylate thereof, and/or
wherein the targeting molecule is negatively charged,
wherein the use involves administration of the dosage in an amount of 0.1-1000 nMole system/kg body weight.

2. Dosage for a use according to claim 1, wherein the dosage is provided in a physiological solution of 1-50 ml.

3. A dosage for a use according to claim 1 and/or 2, wherein the targeting molecule is selected from: HQ4, HQ5, HQ6, HQ7, ICG, CW 800, ZW800, L4, L7, L11, CY3, CY3b, CY3.5, CY5, CY5.5, CY7, Dy 676, Dy 681, Dy 731, Dy 751 and Dy 776; and, is preferably selected from HQ4, HQ5, CW800 and ZW800.

4. A dosage for a use according to one or more of the preceding claims, wherein the agent is:
(a) an imaging compound selected from a group consisting a luminescent compound, a radioactive tracer, an MRI contrast or Spectroscopic agent, a PET agent, a RFA (Radio Frequency Ablation) agent, a SPECT agent, an ultrasound microbubble or contrast agent, opto-acoustic nanoparticles or contrast agent, CT contrast agent, a Raman agent, and combinations thereof; and/or
(b) a therapeutic compound selected from a group consisting of chemo therapeutic agents, photo dynamic compounds, hyperthermic compounds (including photo thermal agents), immune modulating agents, proteins and peptides, nucleic acids (RNA- or DNA-based), medicaments (like antibodies), and combinations thereof; and/or
(c) a release factor for stimulating tissue regeneration, such as growth factors and stem cells.

5. A dosage for a use according to one or more of the preceding claims, wherein the vehicle is selected from a group consisting: a liposome, a dendrimer, a biodegradable particle, a micelle, a nanoparticle, an acoustic dissociable particle, a pH dissociable particle, a light dissociable particle, a heat dissociable particle, a chelating moiety, and combinations thereof.

6. A dosage for a use according to one or more of the preceding claims, wherein the in the body non-reactive molecule being capable of interacting with a counterpart is selected from: a first Click-Chemistry component, a second Click-Chemistry component being complementary to the first Click-Chemistry component, and a component of a self-labelling protein tag such as of Snap-Tag.

7. Dosage for a use according to any of claims 1-6, wherein the dosage comprises 0.5-500 nMole system/kg body weight, preferably 1-250 nMole system/kg body weight, more preferably 2-100 nMole system/kg body weight, such as 5-50 nMole system/kg body weight.

## Patentansprüche

1. Dosierung zur Verwendung beim Nachweis und/oder bei der Behandlung von Krebsarten und/oder Krankheiten, die den nekrotischen Zelltod einschließen, umfassend eine wirksame Menge eines Systems, das eine erste Entität umfasst, die ein Zielmolekül zur Bindung nekrotischer Zellen ist, wobei das Zielmolekül ein Cyanin ist, das nicht aktiviert ist, das in der Lage ist, sich nicht-kovalent an intrazelluläre Proteine zu binden, wenn die Membranintegrität einer Zelle verloren geht, und das nicht mit DNA interagiert,
wobei das System ferner eine zweite Einheit umfasst, die die zu verbindenden Einheiten, beispielsweise durch eine chemische Bindung, ausgewählt aus einem oder mehreren von ihnen, verbindet:
(a) einem Mittel, ausgewählt aus einer bildgebenden Verbindung, einer therapeutischen Verbindung und einem Freisetzungsfaktor;
(b) einem Vehikel, das für den Transport eines Wirkstoffes/des Wirkstoffes wirksam ist; und
(c) ein im Körper nicht reaktives Molekül, das in der Lage ist, mit einem Gegenstück zu interagieren,
wobei das Zielmolekül ein nicht-reaktiver Cyanin-Farbstoff ist, gemäß Abbildung 1 I, II und III, 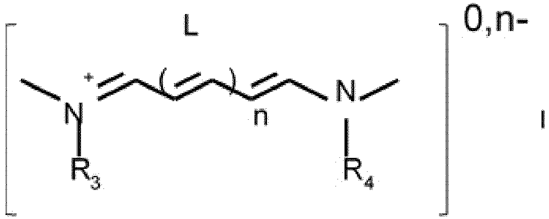 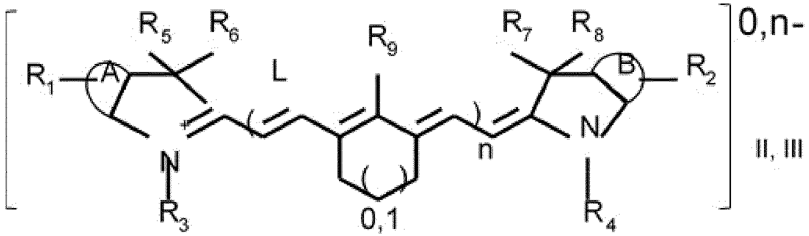 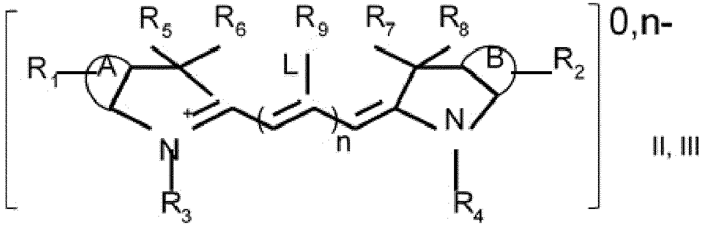
worin n eine ganze Zahl, wie n ∈ [2,10], vorzugsweise n∈ [4,8], ist, die Kette L bis zu n-1 Doppelbindungen, vorzugsweise n/2 Doppelbindungen, aufweist,
wobei die Unterfamilien II und III ein bzw. zwei aromatische Ringsysteme (A,B) umfassen, die durch die gekrümmte(n) Linie(n) C gekennzeichnet sind, und A,B vorzugsweise jeweils einzeln aus Benzol und Naphthalin ausgewählt sind,
worin A, B vorzugsweise jeweils einzeln aus Benzol und Naphthalin ausgewählt sind,
worin weitere Gruppen R₅, R₆, R₇ und R₈ vorhanden sein können, R₅, R₆, R₇ und R₈ vorzugsweise jeweils einzeln ausgewählt sind aus H und Alkyl, wie Methyl, Ethyl und Propyl, vorzugsweise Methyl,
wobei die aromatischen Ringsysteme weitere funktionelle Gruppen R₁, R₂, und/oder Substituenten, umfassen können, R₁, R₂, die vorzugsweise jeweils einzeln aus H, Sulfonat und Sulfonamid ausgewählt sind,
wobei die Kette von alternierenden Einfach- und Doppelbindungen L durch eine oder mehrere teilweise und vollständig gesättigte Ringstrukturen, wie Cyclopenten und Cyclohexen, und Kombinationen davon, wie einen oder mehrere Cyclohexenringe, unterbrochen sein kann, wobei die gesättigte Ringstruktur ferner funktionelle Gruppen Rg umfassen kann,
wobei R₉ ausgewählt ist aus H, AA und BB, wobei R₁₀ ausgewählt ist aus, H, SO₃H, Cl, -N-C=O-(CH₂)_{q}-Y₃ (q=1-6), -(CH2)ᵣ-Y₄ (r=1-6), Y₃ und Y₄ jeweils unabhängig voneinander H, COOH, SO₃H, CN sind,
wobei die Stickstoffatome (N) weitere funktionelle N-Seitengruppen R₃, R₄ umfassen können, wobei R₃, R₄ vorzugsweise jeweils einzeln ausgewählt sind aus -(CH₂)ₘY, wobei Y jeweils einzeln ausgewählt ist aus einer Carbonsäure mit 1-4 Kohlenstoffatomen, einer Sulfonatgruppe, CN, CC und C=C, und Salze davon,
wobei die N-Seitengruppen m Kohlenstoffatome umfassen, wie m ∈[1,10], vorzugsweise m ∈[2,8], stärker bevorzugt m ∈[3,7], am meisten bevorzugt m = 4,5 und 6, noch bevorzugter mindestens eines von m = 4, 5 und 6, vorzugsweise ein m = 6, und das andere m vorzugsweise 4, 5 oder 6,
wobei die N-Seitengruppen eine oder mehrere funktionelle Gruppen an einem dem N entgegengesetzten Ende umfassen, wie eine Carbonsäure mit 1-4 Kohlenstoffatomen, eine Sulfonsäuregruppe und Salze davon, wie Natrium- und Kaliumsalze, wobei am bevorzugtesten die funktionelle Gruppe am Ende eine oder mehrere C-C-Doppelbindungen umfasst, vorzugsweise ein Carboxylat davon, und/oder
wobei das Zielmolekül negativ geladen ist,
wobei die Verwendung die Verabreichung der Dosis in einer Menge von 0,1-1000 nMol System/kg Körpergewicht umfasst.

2. Dosierung für eine Verwendung nach Anspruch 1, wobei die Dosierung in einer physiologischen Lösung von 1-50 ml bereitgestellt wird.

3. Dosierung für eine Verwendung nach Anspruch 1 und/oder 2, wobei das Zielmolekül ausgewählt ist aus: HQ4, HQ5, HQ6, HQ7, ICG, CW 800, ZW800, L4, L7, L11, CY3, CY3b, CY3. 5, CY5, CY5.5, CY7, Dy 676, Dy 681, Dy 731, Dy 751 und Dy 776; und, wird vorzugsweise aus HQ4, HQ5, CW800 und ZW800 ausgewählt.

4. Eine Dosierung für eine Verwendung gemäß einem oder mehreren der vorhergehenden Ansprüche, wobei das Mittel ist:
(a) eine bildgebende Verbindung, ausgewählt aus einer Gruppe, bestehend aus einer lumineszierenden Verbindung, einem radioaktiven Tracer, einem MRI-Kontrast- oder spektroskopischen Mittel, einem PET-Mittel, einem RFA (Radiofrequenz-Ablation)-Mittel, einem SPECT-Mittel, einem Ultraschall-Mikrobläschen- oder Kontrastmittel, opto-akustischen Nanopartikeln oder Kontrastmittel, CT-Kontrastmittel, einem Raman-Mittel und Kombinationen davon; und/oder
(b) eine therapeutische Verbindung, ausgewählt aus einer Gruppe bestehend aus chemotherapeutischen Mitteln, photodynamischen Verbindungen, Hyperthermie-Verbindungen (einschließlich photothermischer Mittel), immunmodulierenden Mitteln, Proteinen und Peptiden, Nukleinsäuren (auf RNA- oder DNA-Basis), Medikamenten (wie Antikörpern) und Kombinationen davon; und/oder (c) einen Freisetzungsfaktor zur Stimulierung der Geweberegeneration, wie Wachstumsfaktoren und Stammzellen.

5. Dosierung für eine Verwendung gemäß einem oder mehreren der vorstehenden Ansprüche, wobei das Vehikel aus einer Gruppe ausgewählt ist, die besteht aus: einem Liposom, einem Dendrimer, einem biologisch abbaubaren Partikel, einer Mizelle, einem Nanopartikel, einem akustisch dissoziierbaren Partikel, einem pH-dissoziierbaren Partikel, einem leicht dissoziierbaren Partikel, einem Wärme-dissoziierbaren Partikel, einer Chelatbildnern Einheit und Kombinationen davon.

6. Dosierung für eine Verwendung gemäß einem oder mehreren der vorhergehenden Ansprüche, wobei der im Körper nicht reaktive Molekül, der in der Lage ist, mit einem Gegenstück zu interagieren, ausgewählt ist aus: einer ersten Click-Chemistry-Komponente, einer zweiten Click-Chemistry-Komponente, die komplementär zu der ersten Click-Chemistry-Komponente ist, und einer Komponente eines selbstmarkierenden Protein-Tags, wie Snap-Tag.

7. Dosierung für eine Verwendung nach einem der Ansprüche 1 bis 6, wobei die Dosierung 0,5 bis 500 nMol System/kg Körpergewicht, vorzugsweise 1 bis 250 nMol System/kg Körpergewicht, noch bevorzugter 2 bis 100 nMol System/kg Körpergewicht, wie z.B. 5 bis 50 nMol system/kg Körpergewicht, umfasst.

## Revendications

1. Dosage destiné à être utilisé dans la détection et/ou le traitement de cancers et/ou de maladies impliquant la mort de cellules nécrotiques comprenant une quantité efficace d'un système comprenant une première entité qui est une molécule de ciblage pour la liaison de cellules nécrotiques, la molécule de ciblage étant une cyanine qui est non activée, qui est capable de se lier de manière non covalente à des protéines intracellulaires lorsque l'intégrité membranaire d'une cellule est perdue, et qui n'interagit pas avec l'ADN,
dans lequel le système comprend en outre une deuxième entité, à laquelle les entités sont reliées, par exemple par une liaison chimique, choisie parmi une ou plusieurs d'entre elles :
(a) un agent choisi parmi un composé d'imagerie, un composé thérapeutique et un facteur de libération ;
(b) un véhicule qui est efficace pour transporter un/un agent ; et
(c) une molécule non réactive dans le corps, capable d'entrer en interaction avec un homologue,
où la molécule cible est un colorant cyanosé non réactif, selon les figures 1 I, II et III, 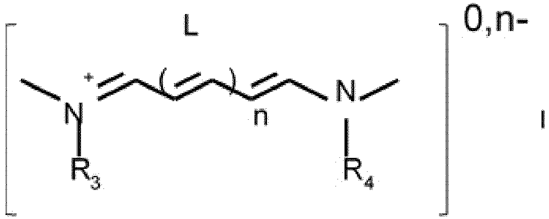 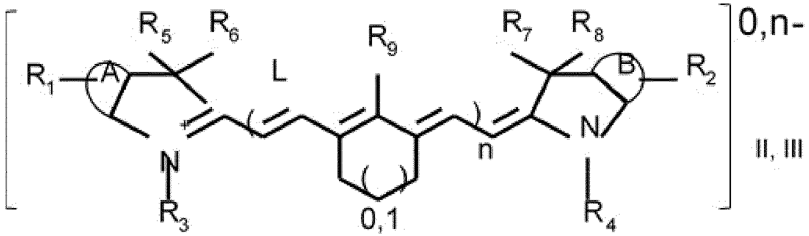 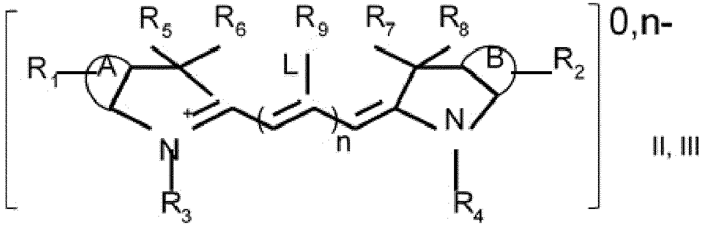
où n est un nombre entier, tel que n ∈ [2,10], de préférence n ∈ [4,8], la chaîne L comporte jusqu'à n-1 doubles liaisons, de préférence n/2 doubles liaisons,
où les sous-familles II et III peuvent comprendre respectivement un et deux systèmes cycliques aromatiques (A, B) représentés par la ligne ou les lignes courbes C,
dans laquelle A et B sont de préférence choisis chacun individuellement parmi le benzène et le naphtalène,
dans laquelle d'autres groupes R₅, R₆, R₇ et R₈ peuvent être présents, R₅, R₆, R₇ et R₈ sont de préférence choisis chacun individuellement parmi H et les groupes alkyle, tels que le méthyle, l'éthyle et le propyle, de préférence le méthyle,
dans laquelle les systèmes cycliques aromatiques peuvent comprendre d'autres groupes fonctionnels R₁, R₂, et/ou des substituants, R₁, R₂, sont choisis de préférence chacun individuellement parmi H, un sulfonate et un sulfonamide,
dans laquelle la chaîne de liaisons simples et doubles alternées L peut être interrompue par une ou plusieurs structures cycliques partiellement et totalement saturées, telles que le cyclopentène et le cyclohexène, et leurs combinaisons, telles qu'un ou plusieurs cycles de cyclohexène, dans laquelle la structure cyclique saturée peut en outre comprendre des groupes fonctionnels Rg, qui sont choisis parmi H, AA et BB, où R₁₀ est choisi parmi H, SO₃H, Cl, -N-C=O-(CH₂)_{q}-Y₃ (q=1-6), -(CH2)ᵣ-Y₄ (r=1-6), Y₃ et Y₄ sont chacun indépendamment l'un de H, COOH, SO₃H et CN,
dans laquelle les atomes d'azote (N) peuvent comprendre d'autres fonctionnels N-groupes latéral R₃, R₄, dans lesquels R₃, R₄ sont de préférence choisis chacun individuellement parmi -(CH₂)ₘY, dans lequel Y est choisi chacun individuellement parmi un acide carboxylique ayant 1-4 atomes de carbone, un groupe sulfonate, CN, CC, et C=C, et leurs sels,
où lesdits N-groupes latéral comprennent m atomes de carbone, tels que m ∈ [1,10], de préférence m ∈ [2,8], plus préférablement m [3,7], plus préférablement m = 4,5, et 6, encore plus préférablement au moins un des m = 4, 5 et 6, de préférence un m = 6, et l'autre m est de préférence 4, 5 ou 6,
dans laquelle lesdits N-groupes latéral comprennent un ou plusieurs groupes fonctionnels sur une extrémité opposée au N, tels qu'un acide carboxylique ayant 1-4 atomes de carbone, un groupe sulfonique, et leurs sels, tels que les sels de sodium et de potassium, de préférence le groupe fonctionnel sur l'extrémité comprend une ou plusieurs doubles liaisons C-C, de préférence un carboxylate de celui-ci, et/ou
où la molécule de ciblage est chargée négativement,
où l'utilisation implique l'administration de la dose dans une quantité de 0,1 à 1000 nMole système/kg de poids corporel.

2. Dosage pour une utilisation selon la revendication 1, dans lequel le dosage est fourni dans une solution physiologique de 1-50 ml.

3. Dosage pour une utilisation selon la revendication 1 et/ou 2, dans lequel la molécule cible est choisie parmi : HQ4, HQ5, HQ6, HQ7, ICG, CW 800, ZW800, L4, L7, L11, CY3, CY3b, CY3. 5, CY5, CY5.5, CY7, Dy 676, Dy 681, Dy 731, Dy 751 et Dy 776 ; et, est de préférence choisi parmi HQ4, HQ5, CW800 et ZW800.

4. Un dosage pour une utilisation selon une ou plusieurs des revendications précédentes, dans lequel l'agent est :
(a) un composé d'imagerie choisi dans le groupe constitué par un composé luminescent, un traceur radioactif, un agent de contraste ou de spectroscopie IRM, un agent TEP, un agent RFA (ablation par radiofréquence), un agent TEMP, un agent de micro-bulles ou de contraste à ultrasons, des nanoparticules ou un agent de contraste optoacoustique, un agent de contraste CT, un agent Raman et leurs combinaisons ; et/ou
(b) un composé thérapeutique choisi dans un groupe constitué d'agents chimio thérapeutiques, de composés photo dynamiques, de composés hyperthermiques (y compris les agents photo thermiques), d'agents de stimulation du système immunitaire, de protéines et de peptides, d'acides nucléiques (à base d'ARN ou d'ADN), de médicaments (comme les anticorps) et de leurs combinaisons ; et/ou (c) un facteur de libération pour stimuler la régénération des tissus, comme les facteurs de croissance et les cellules souches.

5. Dosage pour une utilisation selon une ou plusieurs des revendications précédentes, dans lequel le véhicule est choisi dans un groupe constitué par : un liposome, un dendrimère, une particule biodégradable, une micelle, une nanoparticule, une particule dissociable acoustique, une particule dissociable de pH, une particule dissociable de lumière, une particule dissociable de chaleur, une fraction chélatante, et des combinaisons de celles-ci.

6. Dosage pour une utilisation selon une ou plusieurs des revendications précédentes, dans lequel la molécule non réactive dans le corps, capable d'interagir avec un homologue, est choisie parmi : un premier composant de chimie de clic, un second composant de chimie de clic complémentaire du premier composant de chimie de clic, et un composant d'un marqueur protéique auto-étiquetant tel que Snap-Tag.

7. Dosage pour une utilisation selon l'une des revendications 1-6, dans lequel le dosage comprend 0,5-500 nMole système/kg de poids corporel, de préférence 1-250 nMole système/kg de poids corporel, plus préférablement 2-100 nMole système/kg de poids corporel, tel que 5-50 nMole système/kg de poids corporel.
